# EUROPÄISCHE PATENTSCHRIFT

(11) **EP 1 055 397 B1**
(45) Veröffentlichungstag und Bekanntmachung des Hinweises auf die Patenterteilung: **23.05.2001**
(21) Anmeldenummer: 99108380.9
(22) Anmeldetag: 29.04.1999
(51) Int. Cl.: A61B 17/32

(54) **Medizinisches Instrument zum Präparieren von Gewebe**
Medical instrument for preparing tissue
Instrument médical de préparation de tissus

(43) Veröffentlichungstag der Anmeldung: 29.11.2000
(73) Patentinhaber: Karl Storz GmbH & Co. KG, 78532 Tuttlingen (DE)
(72) Erfinder: Lang, Dieter, 96342 Stockheim (DE); Bacher, Uwe, 78532 Tuttlingen (DE); Anders, Fridolin, 78194 Immendingen (DE)
(74) Vertreter: Heuckeroth, Volker

(56) Entgegenhaltungen:
- DE-A- 4 300 064
- DE-A- 19 520 717
- DE-U- 8 712 835
- US-A- 4 646 745
- US-A- 5 507 772

## Beschreibung

Die Erfindung betrifft ein medizinisches Instrument zum Präparieren von Gewebe im menschlichen oder tierischen Körper nach dem Oberbegriff des Anspruchs 1.

Ein solches medizinisches Instrument ist aus der US 5,507,772 bekannt.

Bei einem Instrument zum Präparieren von Gewebe handelt es sich um ein Instrument zum Abtrennen und/oder Fassen von Gewebe, beispielsweise um eine Stanze, Schere oder Zange.

Derartige Instrumente werden im Rahmen der minimal-invasiven Chirurgie dazu verwendet, Gewebe im menschlichen oder tierischen Körper, üblicherweise unter endoskopischer Sichtkontrolle, abzutrennen. Dazu weisen derartige Instrumente einen lang erstreckten Schaft auf, an dessen distalem Ende zumindest ein bewegliches Werkzeug angeordnet ist, das üblicherweise mit einem weiteren beweglichen oder unbeweglichen Werkzeug am distalen Ende des Schafts zum Abtrennen des Gewebes zusammenwirkt. Zum Betätigen des zumindest einen beweglichen Werkzeuges weisen derartige Instrumente ferner am proximalen Ende des Schafts zumindest ein bewegliches Griffteil auf, das über ein auf Schub und/oder Zug arbeitendes axial bewegliches Kraftübertragungselement kraftschlüssig mit dem zumindest einen beweglichen Werkzeug verbunden ist, so daß eine Bewegung des beweglichen Griffteils vom Kraftübertragungselement in eine Bewegung des beweglichen Werkzeugs übertragen wird.

Insbesondere bei operativen Eingriffen im Hals-Nasen-Ohrenbereich werden Instrumente verwendet, deren Schaft zumindest eine Biegung aufweist, um das oder die Werkzeuge am distalen Ende an schwer zugängliche Stellen, beispielsweise in Nischen der Stirn- oder Kieferhöhle, bringen zu können.

Bei einem Instrument mit einem gebogenen Schaft, insbesondere, wenn der Biegeradius klein ist, stellt sich das Problem, die Kraft vom beweglichen Griffteil über das Kraftübertragungselement durch die Biegung hindurch auf das bewegliche Werkzeug zu übertragen. Dies ist durch den Wunsch nach schlanken, d.h. dünnschaftigen Instrumenten besonders erschwert.

Um sich an die Biegung anpassen zu können, muß das Kraftübertragungselement daher im Bereich der Biegung des Schafts eine Biegeelastizität aufweisen.

Aus der DE 44 44 025 A1 ist beispielsweise ein Kraftübertragungselement für ein medizinisches Instrument in Form eines biegeelastischen Drahtelements offenbart, das sich an eine derartige Biegung anpassen kann. Ein solcher flexibler Draht kann jedoch nur Zugkräfte übertragen, ist jedoch nicht geeignet, Schubkräfte, insbesondere hohe Schubkräfte, zu übertragen. Wird das Kraftübertragungselement nämlich auf Schub belastet, knickt der Draht aus, so daß nur eine geringe oder keine Schubkraft auf das zumindest eine bewegliche Werkzeug übertragen werden kann, um dieses zum Abtrennen von Gewebe zu betätigen.

Da mit derartigen Instrumenten unter anderem auch hartes Gewebe, beispielsweise Knorpel- oder Knochengewebe, abgetrennt werden soll, muß das Kraftübertragungselement daher so beschaffen sein, daß auch sehr hohe Kräfte, insbesondere hohe Schubkräfte, auf das bewegliche Werkzeug übertragen werden können, ohne daß das Kraftübertragungselement dabei ausknickt, wobei die Kraftübertragung andererseits wohlgemerkt über die Biegung des Schafts erfolgen muß, das Kraftübertragungselement sich bei seiner axialen Bewegung also dem Biegungsverlauf anpassen können muß.

Die DE 195 20 717 C2 offenbart zur Lösung dieses Problems ein Rohrschaftinstrument mit einem rohrförmigen Schaft, der eine Biegung aufweist, wobei das Kraftübertragungselement im Innern des Rohrschafts angeordnet ist. Das Kraftübertragungselement ist im gebogenen Bereich des Rohrschafts als Stab aus einem biegeelastischen Material ausgebildet, der an der Innenwand einer ihn umgebenden starren, konzentrisch zum Schaft verlaufenden Hülse anliegt, und dessen Querschnitt im Bereich der Biegung durch eine Anzahl von axialen nebeneinander angeordneten Umfangsnuten bereichsweise reduziert ist. Die zuvor genannte Hülse kann dabei auch durch den Rohrschaft selbst gebildet sein. Die Umfangsnuten sind an dem Stab voll umfänglich ausgebildet. Im Bereich der Umfangsnuten ist der Stab somit drahtförmig verdünnt mit rundem Querschnitt ausgebildet. In den Zwischenbereichen zwischen den Nuten ist der Durchmesser des Stabs nicht verringert, so daß die Außenflächen der Zwischenbereiche als Führungsflächen dienen, um das Kraftübertragungselement im Bereich der Biegung an der Innenwand der Hülse bzw. des Rohrschaftes zu führen.

Mit einem solchen Kraftübertragungselement lassen sich zwar hohe Schubkräfte durch die Biegung hindurch auf das bewegliche Werkzeug übertragen, dennoch kann dieses bekannte Instrument mit dieser Ausgestaltung des Kraftübertragungselements als nachteilig angesehen werden.

Da derartige Instrumente im Rahmen der minimal-invasiven Chirurgie verwendet werden, besteht nämlich eine weitere Anforderung an derartige Instrumente darin, daß ihr Durchmesser im Bereich des Schaftes möglichst klein ist, um das Instrument mit dem Schaft durch eine möglichst kleine Inzision oder natürliche Körperöffnung in den Körper einführen zu können. Bei derartigen miniaturisierten Instrumenten mit einem Schaftdurchmesser von wenigen Millimetern bedeutet dies für das bekannte Instrument, daß das Kraftübertragungselement in den verdünnten Bereichen der Umfangsnuten einen sehr kleinen Durchmesser aufweist, wodurch die Stabilität des Kraftübertragungselements verringert ist, da sich die Materialstärke des Kraftübertragungselements im Bereich der Umfangsnuten auf sehr dünne Drahtabschnitte reduziert. Im Bereich der Umfangsnuten kann es daher bei der Übertragung hoher Schubkräfte oder bei stoßartigen Schubkräften zu einem Knicken, Brechen oder bei Übertragung hoher Zugkräfte zu einem Reißen des Kraftübertragungselements im Bereich der vollumfänglich ausgebildeten Umfangsnuten kommen, wodurch die Betriebssicherheit des bekannten Instruments verringert ist.

Die DE 43 00 064 A1 offenbart eine Gewebestanze mit einem Außenschaft und einem Innenschaft, an dessen distalem Ende eine Öffnung mit einer Schneide vorgesehen ist, die mit einer Gegenschneide am distalen Ende des Außenschaftes so zusammenarbeitet, daß bei Betätigung der Gewebestanze Gewebe, das durch die erwähnte Öffnung in den Innenschaft ragt, durch die gegeneinander bewegten Schneiden abgetrennt wird. Der Innenschaft ist starr ausgebildet, verläuft im proximalen Bereich gerade und geht distalwärts in einen gekrümmten Verlauf über, und der Außenschaft, der das Kraftübertragungselement dieses Instruments bildet, ist zumindest im Bereich dieser Krümmung verformbar. Die Verformbarkeit des Außenschaftes wird dadurch ermöglicht, daß dieser mit Ausnehmungen versehen ist, die sich auf den Radien der Krümmung des Innenschaftes gegenüberliegen. Die Ausnehmungen in Form von radialen Einschnitten belassen lediglich eine schmale Materialbrücke zwischen sich.

Die bereits genannte US 5,507,772 offenbart ebenfalls ein medizinisches Instrument, dessen Schaft eine Biegung aufweist. Das Kraftübertragungselement weist eine Mehrzahl von eingekerbten oder ausgesparten Abschnitten im Bereich der Biegung des Schafts auf. Zwischen den ausgesparten Abschnitten des Kraftübertragungselements sind eine Mehrzahl von Rippen ausgebildet, deren Dicke im wesentlichen der Dicke des übrigen Körpers des Kraftübertragungselements entspricht. Die Rippen sind nur zu einer Seite des Kraftübertragungselements hin ausgebildet, während das Kraftübertragungselement auf der den Rippen gegenüberliegenden Seite im Bereich der Biegung durchgehend flach ausgebildet ist. Das Kraftübertragungselement und dessen Rippen sind in einem länglichen Schlitz innerhalb des Schafts geführt, der in der konkaven Innenseite der Biegung des Schafts vorhanden ist. In einem anderen Ausführungsbeispiel sind die Rippen weggelassen, so daß das Kraftübertragungselement in diesem Fall im Bereich der Biegung als flaches Band ausgebildet ist, das dann aber nicht geführt ist.

Aus der US 4,646,745 ist weiterhin ein medizinisches Klammerinstrument bekannt, das ein in einem Schaft angeordnetes Kraftübertragungselement aufweist, wobei das Kraftübertragungselement in Form eines Flachbandes ausgebildet ist, so daß das Kraftübertragungselement flexibel ist. Das Flachband selbst besteht aus drei Lagen von dünnen Bändern. Im Bereich einer Biegung des Schafts ist das Flachband entlang eines Abstandselements geführt, um zu erreichen, daß das Flachband im Bereich der Biegung seine zentrische Lage im Schaft beibehält, wenn es unter Zugspannung steht, d.h. sich nicht in der Biegung des Schafts gerade zieht. Das Abstandselement ist mit Kugellagern zur Verminderung der Reibung am Kraftübertragungselement versehen.

Der Erfindung liegt die Aufgabe zugrunde, ein medizinisches Instrument der eingangs genannten Art dahingehend weiterzubilden, daß mittels des Kraftübertragungselements über die Biegung des Schaftes hinweg hohe Zug- und/oder Schubkräfte auf das zumindest eine bewegliche Werkzeug übertragen werden können, ohne daß das Kraftübertragungselement dabei ausknickt, und ohne daß im Bereich der Biegung eine Verringerung der Stabilität des Kraftübertragungselements auftritt.

Erfindungsgemäß wird die der Erfindung zugrunde liegende Aufgabe hinsichtlich des eingangs genannten medizinischen Instruments dadurch gelöst, daß das Kraftübertragungselement in Zwischenabschnitten zwischen den abgeflachten Abschnitten umfänglich am Schaft anliegt, wobei sich die Zwischenabschnitte zur konkaven und zur konvexen Innenseite der Biegung des Schafts hin erstrecken.

Bei einem Instrument, das einen Schaft in Form eines Rohrschafts aufweist, und bei dem das Kraftübertragungselement im Innern des Rohrschafts angeordnet ist, ist demnach vorgesehen, das Kraftübertragungselement im Bereich der Biegung zumindest axial abschnittsweise abgeflacht biegeelastisch auszubilden. Abgeflacht bedeutet, daß das Kraftübertragungselement in den abgeflachten Bereichen nicht wie im Stand der Technik vollumfänglich auf Drahtabschnitte verdünnt ist, sondern die Materialreduzierung derart ist, daß sich das Kraftübertragungselement in den abgeflachten Bereichen noch über den gesamten Innendurchmesser des Schafts erstreckt.

In den abgeflachten Bereichen weist das Kraftübertragungselement demnach Stege oder bandartige Abschnitte auf. Die abgeflachten Bereiche können aber auch in sich aus quer zur Längsachse und quer zur Krümmungsebene erstreckenden Einschnitten in dem ansonsten vorzugsweise aus Vollmaterial bestehenden Kraftübertragungselement bestehen. Die abschnittsweise abgeflachte Ausgestaltung des Kraftübertragungselements im Bereich der Biegung führt zu einer Flexibilität des Kraftübertragungselements in der Krümmungsebene, was aber auch ausreichend ist, da die Biegung des Schafts nur eine Biegsamkeit des Kraftübertragungselements in der Krümmungsebene erfordert. Die abschnittsweise abgeflachte Ausgestaltung des Kraftübertragungselements im Bereich der Biegung und die Erstreckung dieser Abschnitte über den Innendurchmesser des Schafts hat jedoch den Vorteil, daß das Kraftübertragungselement trotz der geringeren Materialstärke im Bereich der abgeflachten Abschnitte eine hohe Stabilität besitzt, da sich gerade diese Abschnitte über die gesamte Breite des Schafts erstrecken. Somit wird auch bei einer miniaturisierten Ausgestaltung des Instruments mit sehr geringem Schaftdurchmesser ein im Bereich der Biegung stabiles Kraftübertragungselement geschaffen, im Unterschied zu dem aus der DE 195 20 717 C2 bekannten Instrument, bei dem das Kraftübertragungselement im Bereich der Biegung des Schafts voll umfänglich in Form von Drahtabschnitten verdünnt ist.

Die Zwischenbereiche gewährleisten eine Führung des Kraftübertragungselements im Bereich der Biegung entlang des Schafts mit geringem Spiel und vermeiden, daß das Kraftübertragungselement im Bereich der Biegung in der Krümmungsebene ausknickt.

Dabei ist es weiterhin bevorzugt, wenn die abgeflachten Abschnitte des Kraftübertragungselements in einem materialabtragenden Verfahren aus einem Vollmaterial gebildet sind, wobei die Materialabtragung von zwei gegenüberliegenden Umfangsseiten des Kraftübertragungselements her quer zur Längsrichtung derselben erfolgt.

Hierbei ist von Vorteil, daß das gesamte Kraftübertragungselement aus einem Vollmaterial gebildet werden kann, wobei die abgeflachten Abschnitte beispielsweise durch einen Fräsvorgang in Richtung quer zur Längsachse des Kraftübertragungselements gebildet werden können. Auch die Herstellung des Kraftübertragungselements ist damit gegenüber dem Kraftübertragungselement des bekannten Instruments vereinfacht, da bei letzterem die Umfangsnuten durch einen Drehvorgang gebildet werden müssen.

In einer weiteren bevorzugten Ausgestaltung sind die abgeflachten Abschnitte jeweils axial auf gleicher Höhe liegend von zwei gegenüberliegenden Umfangsseiten des Kraftübertragungselements her ausgebildet.

Hierbei ist von Vorteil, daß sich bezüglich der Längsachse des Kraftübertragungselements mittige Bereiche in Form von Flachbändern ausbilden lassen, für die der maximale Durchmesser des Kraftübertragungselements zur Verfügung steht.

In einer alternativen bevorzugten Ausgestaltung sind die abgeflachten Abschnitte jeweils axial versetzt von zwei gegenüberliegenden Umfangsseiten des Kraftübertragungselements her ausgebildet.

Die Abflachungen sind bei dieser Ausgestaltung in axialer Richtung abwechselnd in der Art eines Mäanders umfänglich versetzt vorhanden, wodurch der Vorteil erreicht wird, daß das Kraftübertragungselement in den demnach einseitig abgeflachten Bereichen im Querschnitt ebenfalls flexibel ist und dennoch mit hoher Stabilität ausgestattet ist.

In einer weiteren bevorzugten Ausgestaltung dieses Instruments ist das Kraftübertragungselement in Bereichen außerhalb der Biegung als massiver Stab aus einem Vollmaterial ausgebildet.

Hierbei ist von Vorteil, daß das Kraftübertragungselement in Bereichen außerhalb der zumindest einen Biegung, insbesondere über sich lang erstreckende gerade Bereiche des Schafts besonders stabil und starr ausgebildet werden kann, wodurch sich das Kraftübertragungselement besonders für die Übertragung hoher Schubkräfte eignet.

In einer weiteren bevorzugten Ausgestaltung des Instruments ist das zumindest eine bewegliche Werkzeug relativ zum Schaft axial beweglich, wobei ein zweites Werkzeug am distalen Ende des Schafts angeordnet ist, wobei das bewegliche Werkzeug und das zweite Werkzeug durch axiale Relativbewegung zueinander in Form einer Stanze zusammenwirken.

Eine solche Ausgestaltung hat insbesondere in sehr beengten Operationsgebieten, beispielsweise in der Stirn- oder Kieferhöhle, den Vorteil, daß die beiden Werkzeuge zum Abtrennen von Gewebe keinen über den Umfang des Schafts hinausgehenden freien Arbeitsradius benötigen, weil beide Werkzeuge lediglich axial in Längsrichtung des Schafts, genauer gesagt in Längsrichtung des distalen Endes des Schafts, relativ zueinander bewegt werden.

Dabei ist es weiterhin bevorzugt, wenn das zweite Werkzeug unbeweglich mit dem Schaft verbunden ist und distal vor dem beweglichen Werkzeug angeordnet ist.

Hierbei ist von Vorteil, daß das zweite unbewegliche Werkzeug die distale Spitze des Schafts bildet und beim Ansetzen dieses unbeweglichen Werkzeugs an dem abzutrennenden Gewebe einen ortsfesten Arbeitspunkt definiert. In diesem Fall arbeitet das Kraftübertragungselement auf Schub, um das bewegliche Werkzeug nach distal gegen das unbewegliche Werkzeug zu schieben, um zwischen dem unbeweglichen Werkzeug und dem beweglichen Werkzeug befindliches Gewebe abzutrennen, und auf Zug, um beide Werkzeuge wieder voneinander zu trennen.

In einer weiteren bevorzugten Ausgestaltung ist das zumindest eine bewegliche Werkzeug gegenüber dem zweiten Werkzeug verdrehgesichert.

Alternativ zu der Ausgestaltung des zumindest einen beweglichen Werkzeuges als Stanzwerkzeug läßt sich die Erfindung jedoch vorteilhaft einsetzen, wenn das zumindest eine bewegliche Werkzeug als um ein Drehgelenk verschwenkbares Maulteil ausgebildet ist, das mit einem zweiten Werkzeug in der Art einer Schere oder Zange zusammenwirkt.

Weitere Vorteile ergeben sich aus der nachfolgenden Beschreibung und der beigefügten Zeichnung.

Es versteht sich, daß die vorstehend genannten und nachstehend noch zu erläuternden Merkmale nicht nur in ihrer jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden hiernach mit Bezug auf diese näher beschrieben. Es zeigen:
- Fig. 1: ein Ausführungsbeispiel eines medizinischen Instruments in Gesamtdarstellung in Seitenansicht;
- Fig. 2: einen Ausschnitt des Instruments in Fig. 1 im Bereich einer Biegung des Schafts teilweise im Längsschnitt, der schematisch ein erstes Ausführungsbeispiel für ein Kraftübertragungselement darstellt;
- Fig. 2a): ein gegenüber Fig. 2 abgewandeltes Ausführungsbeispiel eines Kraftübertragungselements in Alleinstellung;
- Fig. 3: einen Schnitt entlang der Linie XIII-XIII in Fig. 2;

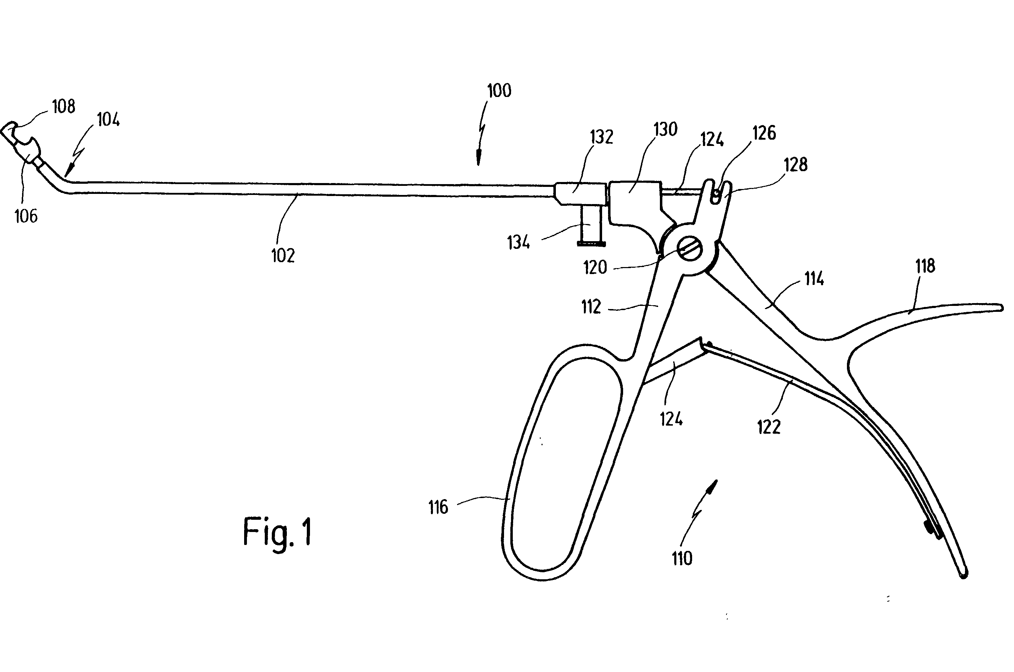

In Fig. 1 ist ein mit dem allgemeinen Bezugszeichen 100 versehenes medizinisches Instrument zum Abtrennen von Gewebe im menschlichen oder tierischen Körper als Ausführungsbeispiel der Erfindung dargestellt.

Das Instrument 100 weist einen lang erstreckten Schaft 102 auf. Der Schaft 102 ist als Rohrschaft ausgebildet. Der Schaft 102 weist eine Biegung 104 auf, die im Bereich des distalen Endes des Schafts 102 angeordnet ist. Die Biegung 104 definiert eine Krümmungsebene, die gleich der Zeichenebene in Fig. 1 ist.

Am distalen Ende des Schafts 102 ist ein bewegliches Werkzeug 106 angeordnet. Am äußersten distalen Ende des Schafts 102 ist ein unbewegliches Werkzeug 108 angeordnet, wobei das bewegliche Werkzeug 106 relativ zu dem unbeweglichen Werkzeug 108 axial verschiebbar ist, um mit dem unbeweglichen Werkzeug 108 zum Abtrennen von Gewebe in der Form einer Stanze zusammenzuwirken.

Am proximalen Ende des Schafts 102 ist eine Handhabe 110 angeordnet. Die Handhabe 110 weist ein bewegliches Griffteil 112 und ein unbewegliches Griffteil 114 aufweist.

Das bewegliche Griffteil 112 weist einen Ring 116 auf, durch den ein, zwei oder drei Finger einer Hand durchgesteckt werden können, um das bewegliche Griffteil 112 zu betätigen. Das unbewegliche Griffteil 114 weist einen nach proximal abstehenden Fortsatz 118 auf, an dem sich die Mulde zwischen Daumen und Zeigefinger beim Greifen der Handhabe 110 abstützt.

Das bewegliche Griffteil 112 und das unbewegliche Griffteil 114 sind über ein Drehgelenk 120 gelenkig miteinander verbunden.

An dem unbeweglichen Griffteil 114 ist eine Blattfeder 122 befestigt, die mit einem Hebel 124 verbunden ist, der an dem beweglichen Griffteil 112 angelenkt ist. Die Blattfeder 122 spannt im Zusammenwirken mit dem Hebel 124 das bewegliche Griffteil 112 in die in Fig. 1 dargestellte, von dem unbeweglichen Griffteil 114 beabstandete Offenstellung vor.

Das bewegliche Werkzeug 106 ist mit dem beweglichen Griffteil 112 durch ein Kraftübertragungselement 124 kraftschlüssig verbunden, das sich längs des Schafts 102 erstreckt und in diesem angeordnet ist. Das Kraftübertragungselement 124 ist relativ zu dem Schaft 102 axial beweglich.

Ein äußerstes proximales Ende des Kraftübertragungselements 124 weist einen Zapfen 126 auf, der sich quer zur Längsrichtung des Kraftübertragungselements 124 erstreckt und in einen Gabelabschnitt 128 des beweglichen Griffteils 112 eingehängt ist.

Das Kraftübertragungselement 124 ist durch ein fest mit dem unbeweglichen Griffteil 114 verbundenes Rohrstück 130 relativ zu diesem beweglich durchgeführt, an dem der Schaft 112 über ein weiteres Rohrstück 132 unbeweglich befestigt ist. An dem Rohrstück 132 ist weiterhin ein Anschluß 134 zum Anschließen einer nicht dargestellten Spülleitung angeordnet, um Spülflüssigkeit in den Schaft 102 einzuleiten, die dann im Schaft 102 zum distalen Ende des Schafts 102 hin geleitet wird, wo sie aus dem Schaft 102 in das Operationsgebiet austritt.

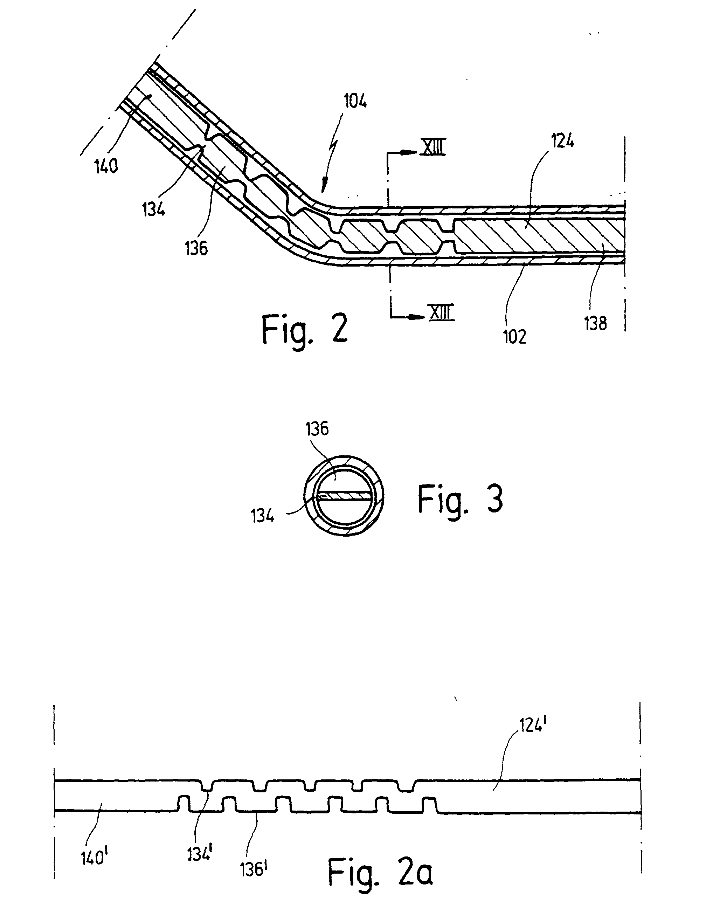

Gemäß Fig. 2 und 3 ist das Kraftübertragungselement 124 im Bereich der Biegung 104 abschnittsweise abgeflacht, und zwar an einer Mehrzahl von abgeflachten Abschnitten 134, von denen das Kraftübertragungselement 124 im in Fig. 2 gezeigten Ausführungsbeispiel insgesamt sechs aufweist.

In den Abschnitten 134 ist das Kraftübertragungselement 124 quer zur Krümmungsebene abgeflacht, so daß das Kraftübertragungselement 124 in der Krümmungsebene der Biegung 104 flexibel ist, und sich an den gekrümmten Verlauf des Schafts 102 im Bereich der Biegung 104 anpassen kann.

In Zwischenabschnitten 136, die aus Vollmaterial bestehen, liegt das Kraftübertragungselement 124 umfänglich am Schaft 102 an.

Die Zwischenabschnitte 136 sind in Form von zylindrischen Körpern ausgebildet, wobei die am Schaft 102 anliegenden Umfangsseiten der Zwischenabschnitte 136 in Längsrichtung leicht konvex ausgebildet sind, so daß jeder der Zwischenabschnitte 136 nur linienförmig an dem Schaft 102 anliegt, wodurch die Reibung der Zwischenabschnitte 136 am Schaft 102 minimiert wird.

Während sich das Kraftübertragungselement 124 im Bereich der Zwischenabschnitte 136 in Richtung quer zur Krümmungsebene, d.h. quer zur Zeichenebene in Fig. 2 über die Breite des Schafts 102 erstreckt, gilt dies auch für die abgeflachten Abschnitte 134, in denen sich das Kraftübertragungselement 124 ebenfalls über den Innendurchmesser des Schafts 102 erstreckt, wie aus Fig. 3 hervorgeht. Die abgeflachten Abschnitte 134 bilden somit Abschnitte, in denen das Kraftübertragungselement 124 abschnittsweise als flaches Band ausgebildet ist.

Die axiale Länge der abgeflachten Abschnitte 134 ist dabei klein gegen die axiale Länge der Zwischenabschnitte 136. Die Abschnitte 134 können sich auf kurze Einschnitte, die im Längsschnitt des Kraftübertragungselements etwa V-förmig ausgebildet sind, reduzieren. Es können jedoch auch andere Längenverhältnisse zwischen den Abschnitten 134 und 136 gewählt werden.

Durch die Ausgestaltung des Kraftübertragungselements 124 weist dieses lediglich eine Flexibilität in Richtung der Krümmung des Schafts 102 auf, ist jedoch in Richtung quer zur Krümmungsebene der Biegung 104 nicht flexibel, sondern starr.

Im Bereich außerhalb der Biegung 104 ist das Kraftübertragungselement 124 als Stab 138 ausgebildet, der einen runden Querschnitt aufweist und in dem Schaft 102 an diesem anliegend geführt ist.

Ein distaler Abschnitt 140 des Kraftübertragungselements 124 ist in geeigneter Weise mit dem beweglichen Werkzeug 106 kraftschlüssig verbunden, um dieses bei Betätigung des beweglichen Griffteils 112 relativ zu dem unbeweglichen Werkzeug 108 zu bewegen.

Die abgeflachten Abschnitte 134 des Kraftübertragungselements 124 sind dadurch gebildet, daß das als Stab vorliegende Kraftübertragungselement 124 in einem materialabtragenden Verfahren von zwei gegenüber liegenden Umfangsseiten des Kraftübertragungselements 124 her, beispielsweise durch Fräsen, beidseitig abgeflacht wird, wobei die Abflachungen an jeweils axial gleicher Position ausgebildet sind.

In Fig. 2a) ist dagegen ein Kraftübertragungselement 124' dargestellt, bei dem abgeflachte Abschnitte 134' jeweils von zwei gegenüberliegenden Umfangsseiten des Kraftübertragungselements 124' her, jedoch axial in der Art eines Mäanders versetzt, vorhanden sind. Zwischenabschnitte 136' liegen dementsprechend ebenfalls axial versetzt zueinander. Auch bei dieser Ausgestaltung erstreckt sich das Kraftübertragungselement 124' in den abgeflachten Abschnitten 134' über den gesamten inneren Durchmesser des hier nicht dargestellten Schafts.

## Patentansprüche

1. Medizinisches Instrument zum Präparieren von Gewebe im menschlichen oder tierischen Körper, mit einem Schaft (102), mit zumindest einem beweglichen Werkzeug (106) an einem distalen Ende des Schafts (102), mit zumindest einem beweglichen Griffteil (112) an einem proximalen Ende des Schafts (102), und mit einem sich längs des Schafts (102) erstreckenden, relativ zu diesem beweglichen auf Schub und/oder Zug arbeitenden Kraftübertragungselement (124; 124'), dessen proximales Ende mit dem zumindest einen beweglichen Griffteil (112) und dessen distales Ende mit dem zumindest einen beweglichen Werkzeug (106) kraftschlüssig verbunden ist, wobei der Schaft (102) an zumindest einer Stelle zwischen dem distalen Ende und dem proximalen Ende eine Biegung (104) aufweist, wobei das Kraftübertragungselement (124; 124') zumindest im Bereich der Biegung (104) zumindest abschnittsweise quer zu einer Krümmungsebene der Biegung (104) abgeflacht biegeelastisch ausgebildet ist, und wobei sich das Kraftübertragungselement (124; 124') im Bereich der Biegung (104) in Richtung quer zur Krümmungsebene axial durchgehend zumindest über den Innendurchmesser des Schafts (102) erstreckt, und wobei das Kraftübertragungselement (124; 124') im Bereich der Biegung eine Mehrzahl von axial beabstandeten abgeflachten Abschnitten (134; 134') aufweist, dadurch gekennzeichnet, daß das Kraftübertragungselement (124; 124') in Zwischenabschnitten (136; 136') zwischen diesen Abschnitten (134) umfänglich am Schaft (102) anliegt, wobei sich die Zwischenabschnitte (136; 136') zur konkaven und zur konvexen Innenseite der Biegung (104) des Schafts (102) hin erstrecken.

2. Instrument nach Anspruch 1, dadurch gekennzeichnet, daß die abgeflachten Abschnitte (134; 134') des Kraftübertragungselements (124; 124') in einem materialabtragenden Verfahren aus einem Vollmaterial gebildet sind, wobei die Materialabtragung von zwei gegenüberliegenden Umfangsseiten des Kraftübertragungselements (124; 124') her quer zur Längsrichtung des Kraftübertragungselements (124; 124') erfolgt.

3. Instrument nach Anspruch 1 oder 2, dadurch gekennzeichnet, daß die abgeflachten Abschnitte (134) jeweils axial auf gleicher Höhe liegend von zwei gegenüberliegenden Umfangsseiten des Kraftübertragungselements (124) her ausgebildet sind.

4. Instrument nach Anspruch 1 oder 2, dadurch gekennzeichnet, daß die abgeflachten Abschnitte (134') jeweils axial versetzt von zwei gegenüberliegenden Umfangsseiten des Kraftübertragungselements (124') her ausgebildet sind.

5. Instrument nach einem der Ansprüche 1 bis 4, dadurch gekennzeichnet, daß das Kraftübertragungselement (124; 124') in Bereichen außerhalb der Biegung (104) als massiver Stab (138) aus einem Vollmaterial ausgebildet ist.

6. Instrument nach einem der Ansprüche 1 bis 5, dadurch gekennzeichnet, daß das zumindest eine bewegliche Werkzeug (106) relativ zum Schaft (102) axial beweglich ist, wobei ein zweites Werkzeug (108) am distalen Ende des Schafts (102) angeordnet ist, wobei das bewegliche Werkzeug (106) und das zweite Werkzeug (108) durch axiale Relativbewegung zueinander in Form einer Stanze zusammenwirken.

7. Instrument nach Anspruch 6, dadurch gekennzeichnet, daß das zweite Werkzeug (108) unbeweglich mit dem Schaft (102) verbunden ist und distal vor dem beweglichen Werkzeug (106) angeordnet ist.

8. Instrument nach Anspruch 6 oder 7, dadurch gekennzeichnet, daß das bewegliche Werkzeug (106) relativ zum zweiten Werkzeug (108) verdrehgesichert ist.

9. Instrument nach einem der Ansprüche 1 bis 5, dadurch gekennzeichnet, daß das zumindest eine bewegliche Werkzeug als um ein Drehgelenk verschwenkbares Maulteil ausgebildet ist, das mit einem zweiten Werkzeug in der Art einer Schere oder Zange zusammenwirkt.

## Claims

1. Medical instrument for dissecting tissue in the human or animal body, comprising a shaft (102), at least one movable tool (106) at a distal end of the shaft (102), at least one movable grip element (112) at a proximal end of the shaft (102), and a force transmission element (124; 124') extending along the shaft (102) and being movable relative to the latter and acting on push and/or pull forces, whose proximal end is non-positively joined to the at least one movable grip element (112), and whose distal end is non-positively joined to the at least one movable tool (106), the shaft (102) being provided with a bend (104) at at least one point between the distal end and the proximal end, wherein, at least in the area of the bend (104), the force transmission element (124; 124') is at least sectionally and transversely to the plane of curvature of the bend (104) configured flattened and flexible, and the force transmission element (124; 124') extends continuously in axial direction in the area of the bend transversely to the plane of curvature at least over the inner diameter of the shaft (102), and wherein the force transmission element (124; 124') comprises a plurality of axially spaced flattened sections (134; 134') in the area of the bend, characterized in that the force transmission element (124; 124') is in contact by its circumference with the shaft (102) in intermediate sections (136; 136') between those sections (134), wherein the intermediate sections (136; 136') extend to the concave inner side and to the convex inner side of the bend (104) of the shaft (102).

2. The instrument of claim 1, characterized in that the flattened sections (134; 134') of the force transmission element (124; 124') are formed from solid material by a machining process, the material being removed from two opposite circumferential sides of the force transmission element (124; 124'), transversely to the longitudinal direction of the force transmission element (124; 124')

3. The instrument of claim 1 or 2, characterized in that each of the flattened sections (134) is formed at the same axial position from two opposite sides of the circumference of the force transmission element (124).

4. The instrument of claim 1 or 2, characterized in that the flattened sections (134') are formed in axially offset arrangement from opposite sides of the circumference of the force transfer element (124').

5. The instrument of one of claims 1 through 4, characterized in that the force transmission element (124; 124') is configured as a solid bar (138) made of solid material, in areas outside the bend (104).

6. The instrument of one of claims 1 through 5, characterized in that the at least one movable tool (106) is axially movable relative to the shaft (102), and a second tool (108) is arranged at the distal end of the shaft (102), the movable tool (106) and the second tool (108) acting together in the way of a punch if moved axially one relative to the other.

7. The instrument of claim 6, characterized in that the second tool (108) is connected with the shaft (102) immovably, and is arranged distally before the movable tool (106).

8. The instrument of claim 6 or claim 7, characterized in that the movable tool (106) is protected from rotation relative to the second tool (108).

9. The instrument of one of claims 1 through 5, characterized in that the at least one movable tool is configured as a jaw part which can be pivoted about a hinge joint and which coacts with a second tool in the manner of a pair of scissors or a forceps.

## Revendications

1. Instrument médical pour la préparation de tissus dans le corps humain ou animal, comportant une tige (102), comportant au moins un outil (106) déplaçable à une extrémité distale de la tige (102), comportant au moins une poignée (112) déplaçable à une extrémité proximale de la tige (102), et comportant un élément de transmission de forces (124 ; 124') qui s'étend le long de la tige (102), qui est déplaçable par rapport à celle-ci et qui travaille en poussée et/ou en traction et dont l'extrémité proximale est reliée à force à la ou aux poignée(s) (112) déplaçable(s) et dont l'extrémité distale est reliée à force à l'outil ou aux outils (106) déplaçables, dans lequel la tige (102) présente un coude (104) en un emplacement au moins entre l'extrémité distale et l'extrémité proximale, dans lequel l'élément de transmission de forces (124 ; 124') est aplati et élastique en flexion au moins dans la zone du coude (104), au moins par endroits transversalement à un plan de courbure du coude (104), et dans lequel l'élément de transmission de forces (124 ; 124') s'étend axialement en continu, au moins sur le diamètre intérieur de la tige (102), dans la zone du coude (104), dans une direction perpendiculaire au plan de courbure, et dans lequel l'élément de transmission de forces (124 ; 124') présente, dans la zone du coude, une pluralité de segments (134; 134') aplatis, espacés axialement, caractérisé en ce que l'élément de transmission de forces (124; 124') s'applique périphériquement contre la tige (102), dans des segments intermédiaires (136; 136') entre ces segments (134), les segments intermédiaires (136; 136') s'étendant vers le côté intérieur concave et le côté intérieur convexe du coude (104) de la tige (102).

2. Instrument selon la revendication 1, caractérisé en ce que les segments aplatis (134 ; 134') de l'élément de transmission de forces (124 ; 124') sont formés par un procédé d'enlèvement de matière dans une matière massive, l'enlèvement de matière s'effectuant à partir de deux côtés périphériques opposés de l'élément de transmission de forces (124; 124'), perpendiculairement à la direction longitudinale de l'élément de transmission de forces (124. 124').

3. Instrument selon la revendication 1 ou 2, caractérisé en ce que les segments aplatis (134) disposés axialement à même hauteur sont formés chacun à partir de deux côtés périphériques opposés de l'élément de transmission de forces (124).

4. Instrument selon la revendication 1 ou 2, caractérisé en ce que les segments aplatis (134'), décalés axialement, sont formés à partir de deux côtés périphériques opposés de l'élément de transmission (124').

5. Instrument selon l'une des revendications 1 à 4, caractérisé en ce que l'élément de transmission de forces (124; 124') est formé dans des zones extérieures au coude (104), sous la forme d'une barre massive (138) faite d'une matière massive.

6. Instrument selon l'une des revendications 1 à 5, caractérisé en ce que l'outil ou les outils (106) déplaçables, sont déplaçables axialement par rapport à la tige (102), un deuxième outil (108) étant disposé à l'extrémité distale de la tige (102), l'outil (106) déplaçable et le deuxième outil (108) coopérant par déplacement relatif axial, sous la forme d'une presse à découper.

7. Instrument selon la revendication 6, caractérisé en ce que le deuxième outil (108) est relié non déplaçable à la tige (102) et est disposé côté distal devant l'outil (106) déplaçable.

8. Instrument selon la revendication 6 ou 7, caractérisé en ce que l'outil (106) déplaçable est bloqué en rotation par rapport au deuxième outil (108).

9. Instrument selon l'une des revendications 1 à 5, caractérisé en ce que l'outil ou les outils déplaçables est ou sont réalisés à la manière d'une mâchoire pouvant pivoter autour d'une articulation de rotation et coopèrent avec un deuxième outil à la manière de ciseaux ou d'une pince.
